# EUROPEAN PATENT SPECIFICATION

(11) **EP 4 342 513 B1**
(45) Date of publication and mention of the grant of the patent: **29.07.2026**
(21) Application number: 22844883.3
(22) Date of filing: 02.04.2022
(51) Int. Cl.: A61M 16/04

(54) **TUBE-WALL-FREE RIGHT-SIDED DOUBLE-LUMEN BRONCHIAL CATHETER**
ROHRWANDFREIER RECHTSSEITIGER BRONCHIALKATHETER MIT DOPPELLUMEN
CATHÉTER BRONCHIQUE À DOUBLE LUMIÈRE CÔTÉ DROIT SANS PAROI DE TUBE

(30) Priority: 18.07.2021 CN 202110810230; 18.07.2021 CN 202121628179 U
(43) Date of publication of application: 27.03.2024
(73) Proprietor: Beijing BYA Innovation Medical Technology Co., Ltd., Beijing (CN)
(72) Inventor: Jianwen Zhou, Shanghai (CN)
(74) Representative: Nederlandsch Octrooibureau
(86) International application number: PCT/CN2022/084957
(87) International publication number: WO 2023/000711

(56) References cited:
- CN-A- 108 187 206
- CN-A- 110 448 775
- CN-A- 113 384 791
- CN-U- 201 643 306
- CN-U- 204 543 184
- CN-U- 205 181 954
- CN-U- 209 713 913
- CN-U- 211 068 564
- US-A1- 2016 157 708
- US-A1- 2017 043 111
- US-A1- 2021 052 840
- US-B2- 9 687 621
- TSAI K M: "Lung isolation update", SEMINARS IN ANESTHESIA, SAUNDERS, CO, NEW YORK, NY, US, vol. 22, no. 2, 1 June 2003 (2003-06-01), pages 88 - 105, XP004903239, ISSN: 0277-0326, DOI: 10.1016/S0277-0326(03)00008-4
- CERFOLIO ROBERT J ET AL: "Decreasing Time to Place and Teach Double-Lumen Endotracheal Intubation: Engaging Anesthesia in Lean", THE ANNALS OF THORACIC SURGERY, ELSEVIER, vol. 106, no. 5, 23 July 2018 (2018-07-23), pages 1512 - 1518, XP085510467, ISSN: 0003-4975, DOI: 10.1016/J.ATHORACSUR.2018.06.023

## Description

### Technical Field

The present invention relates to the technical field of medical equipment, specifically a tube-wall-free right-sided double-lumen bronchial catheter.

### Technical backgrounds

The double-lumen bronchial catheter(DLT) can isolate the left lung from the other side, ensuring one-lung ventilation, providing good visual fields for the surgeon and preventing the healthy lung from being infected with blood and secretion of the affected lung, etc., which makes it the most frequently used ventilation tool in thoracic and cardiac surgery.

Currently, DLT frequently used are mainly categorized into left double-lumen bronchial catheter(LDLT) and right double-lumen bronchial catheter(RDLT). When using DLT for pulmonary isolation ventilation, it is preferable to place DLT into the non-operative side of the lung in order not to interfere with the surgical operation. To be specific, LDLT is used in right-sided thoracic surgery and RDLT is used in left-sided thoracic surgery.

Due to the anatomical structure of human airway, the left main bronchus is slender and long while the right one is relatively thick and short, and the bronchial opening of superior lobe of right lung is more variable and at a higher position. Therefore, sufficient ventilation can be achieved by using a LDLT for one-lung ventilation(OLV).

In comparison with LDLT, RDLT has side holes in the wall below the bronchial cuff to aid in ventilation of right upper lobe. In clinical practice, when using RDLT for OLV, anesthesiologists have to spend time adjusting the position and direction of RDLT to ensure adequate ventilation of upper lobe. However, poor catheter alignment, due to the high variability of the opening position of bronchial catheter in the upper lobe, and the blockage of the side holes with sputum, blood and other secretion may result in poor ventilation of the right lung, Which may cause oxygenation in the right lobe. It is more likely to cause a series of complications such as insufficient ventilation oxygenation, right upper lobe atelectasis, and severe imbalance of ventilation blood flow ratio.

Therefore, it is rather necessary to guarantee the highly efficient ventilation by using a RDLT that can ensure bilateral lung isolation and OLV of right lung.

US2017043111A1 provides improved dual lumen endobronchial tube devices. The dual lumen endobronchial tube devices feature a universal design for left or right mainstem bronchus insertion. The dual lumen endobronchial tube devices also feature enhanced balloon cuff designs to minimize dislodgement while maintaining proper airway sealing. The present invention also includes water activated lubricious coating inside the shaft to reduce friction during insertion of a bronchoscope into the airway. The present invention also provides improved double clamps that prevent the accidental clamping of both tubes of a Y-adapter.

US2021052840A1 discloses a tracheal tube inserted through the trachea and bronchi of a subject, comprising: a tube body having a through hole on an outer circumferential surface thereof, a bronchial cuff provided on an outer circumferential surface of the tube body and pressing an inner circumferential surface of the bronchus;wherein the bronchial cuff is spaced apart in the axial direction of the tube body and forms a plurality of ventilation spaces;wherein the ventilation spaces are in communication with each other;wherein the through hole communicate with the ventilation space.

CN204543184U provides a two-chamber endotracheal tube relates to clinical medicine equipment field, include: the main part pipe of ventilating, the connection is in the person in charge that the main part was ventilated on the pipe overlaps bag inflation valve and bronchus cover bag inflation valve, the cover is established on the main part breather pipe and with the person in charge who is responsible for cover bag inflation valve intercommunication overlaps the bag, the cover is established on the main part breather pipe and with the bronchus cover bag of bronchus cover bag inflation valve intercommunication, the pipe is ventilated to the main part is the two-chamber pipe, in the said two-chamber pipe, be provided with a plurality of side openings of ventilating on the end of its mesobronchus chamber pipe, can guarantee the effective ventilatory volume of patient lung, reduce pipe dislocation rate to prevent oxygen-deficient emergence, and save the intubate time, reduce repeatedly the damage that leads to the fact the debugging location to patient's trachea mucous membrane.

CN211068564U discloses an improved right double-cavity bronchial catheter. The catheter comprises a right catheter and a left catheter, the right guide pipe and the left guide pipe are arranged in parallel; the total length of the right guide pipe and the left guide pipe ranges from 15 cm to 42 cm. A bronchial cuff inflation tube is arranged at the upper end of one side, far away from the left catheter, of the right catheter; a bronchial cuff inflation inlet is formed in the end, away from the right catheter, of the bronchial cuff inflation tube, a tracheal cuff inflation tube is arrangedat the upper end of the side, away from the right catheter, of the left catheter, and a tracheal cuff inflation inlet is formed in the end, away from the left catheter, of the tracheal cuff inflationtube. The double-cavity ventilation device is simple in structure and reasonable in design, avoids the contradiction between double-lung isolation and insufficient ventilation in the single-lung ventilation process, benefits surgical operation and anesthesia management, is easy to operate and high in clinical practicability, simplifies the manufacturing process, and can basically replace right-side double-cavity ventilation of an existing airway management medical instrument.

### Contents of the Invention

The present invention breaks through today's technical bottleneck, and allows the RDLT to perform double-lung isolation, OLV, and at the same time ensure the ventilation effect to the greatest extent.

The invention is set out in the appended set of claims

In comparison with current techniques the present invention breaks through the constraints of the "tube" in the tracheal catheter, and "wall-less" design in bronchial catheter maximally guarantees the normal ventilation of the upper lobe of the right lung and ensure optimal ventilation of the right lung in single-lung ventilation, various measures are taken to prevent issues such as misalignment of side holes ,obstruction of side holes by secretions, and blocking of the tracheal opening by the tube wall. These measures aim to minimize ventilation impairment in the right upper lung, ensuring proper airflow and reducing the risk of complications associated with inadequate ventilation. Additionally, by minimizing the time required for adjusting the tube and the potential for airway injuries, this approach contributes to an overall improvement in the efficiency and safety of single-lung ventilation.

### Description of drawings

Fig. 1 shows a schematic diagram of the structure of a tube-wall-free bronchial catheter body;
Fig. 2 shows a schematic diagram of the overall structure of a tube-wall-free right-sided double-lumen bronchial catheter.

In attached drawings: 1.1bronchial cuff; 1.2bronchial strut; 1 .3 annular opening; 2 .1 main tracheal catheter; 2 .2 right bronchial catheter; 2 .3 main tracheal cuff; 2.4 a main tracheal catheter opening; 2 .5 bronchial cuff inflator; 2 .6 main tracheal cuff connection tube 2.7 bronchial cuff connection tube

### Specific embodiments

The present invention will be further described below with attached drawings, the description doesn't constitute a limitation of the present invention.

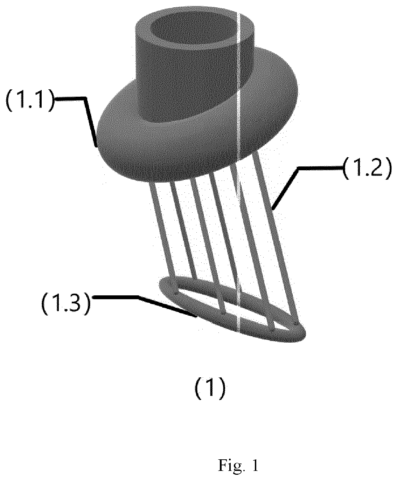

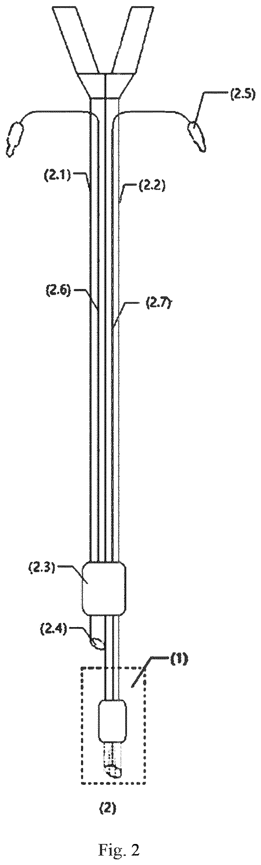

Referring to the fig. 1 and fig. 2, the tube-wall-free right-sided double-lumen bronchial catheter tube consists primarily of the tube-wall-free bronchial catheter body 1 and double-lumen ventilation catheter body 2. The tube-wall-free bronchial catheter body 1 comprises the bronchial cuff 1.1, several bronchial struts 1.2 and the annular opening 1.3. The bronchial catheter of the lower section of the bronchial cuff 1.1 in the tubeless wall type bronchial catheter 1 has no tubular wall structure, and the bronchial catheter of the tubeless wall structure is connected to the inner wall of the bronchial cuff 1.1 via the upper end of a plurality of bronchial struts 1.2, the lower end of a plurality of bronchial struts 1.2 connected to the annular opening 1.3, bronchial struts 1.2 connected to each other without wall structure. The mentioned double-lumen ventilation catheter body 2 consists of the main tracheal catheter 2.1, the right bronchial catheter 2.2, the main tracheal cuff 2.3, the main tracheal catheter opening 2.4, the main tracheal cuff inflation device, the bronchial cuff inflation device 2.5, among them, the lower end of the right bronchial catheter connected to the tube-wall-free bronchial catheter body 1. Double-lumen ventilation catheter body 2 is a tube with two-lumen structure, with the left side being the main tracheal catheter 2.1 and the right side being the right bronchial catheter 2.2. The main tracheal cuff 2.3 is in the lower section of the double-lumen ventilation catheter body 2, and below the main tracheal cuff 2.3 is the main tracheal catheter opening 2.4, which is a beveled opening facing downward to the left. The main tracheal cuff inflation device is connected to the main tracheal cuff 2.3 via the main tracheal cuff connection tube 2.6 parallel to the double-lumen ventilation catheter body 2. The bronchial cuff inflation device 2.5 is connected to the bronchial cuff 1.1 via the bronchial cuff connection tube 2.7 parallel to the double-lumen ventilation catheter body 2.

The main tracheal cuff connection tube 2.6 and the bronchial cuff connection tube 2.7 are thin tubes.

In the specific implementation of the present invention, when the a tube-wall-free bronchial catheter body enters the right main bronchus and the bronchial cuff is flush with the level of the tracheal bulge of the bronchial cuff, the main tracheal catheter cuff and the bronchial cuff are inflated, and during the process of OLV, there is no need for adjusting the catheter direction and position, and the tube-wall-free structure of the bronchial catheter can realize the complete opening of the right upper lobe of the lung to guarantee the optimal ventilating effect of OLV.

On the basis of the traditional RDLT, the present invention abandons the previous structure of the right bronchial catheter, breaking through the barrier of the "tube", and the right bronchial catheter adopts the structure of the tubeless wall, which changes the method of relying on the wall, the lumen, and the structure of the side hole in the traditional catheter to carry out ventilation, and radically solves the problem of misalignment, secretion blockage, and the problem of the side hole, which are the most common problems of the right lung, and the most common problems of the right lung. It can fundamentally solve the problem of poor ventilation caused by misalignment of side holes, obstruction of side holes by secretions, and blocking of the tracheal opening by the tube wall, which not only saves the anesthesiologist's time for adjusting the positioning, but also maximally protects the patient's OLV effect and avoids various complications caused by poor ventilation due to the defect of the catheter. The design of the present invention of the tubeless wall bronchial catheter is simple, but it is easy to manufacture and has remarkable effects and strong Clinical practical value. If it can be popularized and applied, it will play a significant clinical value.

The foregoing is only a preferred embodiment of the present invention, and it should be noted that a number of improvements and additions, without departing from the present invention, can be made by ordinary technical personnel, which shall also be considered to be within the scope of protection of the present invention.

## Claims

1. The tube-wall-free right-sided double-lumen bronchial comprising:
- a double-lumen ventilation catheter body (2) comprising a main tracheal catheter (2.1) and a right bronchial catheter (2.2),
- a main tracheal cuff (2.3), an
- a tube-wall-free bronchial catheter body (1) consisting of
- - a bronchial cuff (1.1)
- - bronchial struts (1.2), an
- - an annular opening (1.3)
wherein the lower end of the right bronchial catheter (2.2) is connected to the tube-wall-free bronchial catheter body (1)
wherein a bronchial catheter of a lower section of the bronchial cuff (1.1) has tube-wall-free structure and is connected to an inner wall of the bronchial cuff (1. 1) via upper ends of the bronchial struts (1.2), and
wherein lower ends of the bronchial struts (1.2) are connected to the annular opening (1.3).

2. The tube-wall-free right-sided double-lumen bronchial catheter as defined in claim 1 further comprising a main tracheal cuff inflation device, and a bronchial cuff inflation device (2.5).

3. The tube-wall-free right-sided double-lumen bronchial catheter as defined in claim 1 or 2, wherein the double-lumen ventilation catheter body (2) is a tube with two-lumen structure, with the left side being the main tracheal catheter (2.1) and the right side being the right bronchial catheter (2.2).

4. The tube-wall-free right-sided double-lumen bronchial catheter as defined in claim 1 or 2, wherein the main tracheal cuff (2.3) is in the lower section of the double-lumen ventilation catheter body (2), the main tracheal catheter opening (2.4) is below the main tracheal cuff (2.3), and the main tracheal catheter opening (2.4) is a beveled opening facing downward to the left

5. The tube-wall-free right-sided double-lumen bronchial catheter as defined in claim 2, wherein the main tracheal cuff inflation device is connected to the main tracheal cuff (2.3) via a main tracheal cuff connection tube (2.6) parallel to the double-lumen ventilation catheter body (2).

6. The tube-wall-free right-sided double-lumen bronchial catheter as defined in claim 2, wherein the bronchial cuff inflation device (2.5) is connected to the bronchial cuff (1.1) via a bronchial cuff connection tube (2.7) parallel to the double-lumen ventilation catheter body (2).

## Patentansprüche

1. Schlauchwandfreier rechtsseitiger Doppellumen-Bronchialkatheter, der Folgendes umfasst:
- einen Doppellumen-Beatmungskatheterkörper (2), der einen Haupttrachealkatheter (2.1) und einen rechten Bronchialkatheter (2.2) umfasst,
- eine Haupttrachealmanschette (2.3), und
- einen schlauchwandfreien Bronchialkatheterkörper (1), der aus Folgendem besteht:
- einer Bronchialmanschette (1.1)
- Bronchialstreben (1.2), und
- einer ringförmigen Öffnung (1.3)
wobei das untere Ende des rechten Bronchialkatheters (2.2) mit dem schlauchwandfreien Bronchialkatheterkörper (1) verbunden ist,
wobei ein Bronchialkatheter eines unteren Abschnitts der Bronchialmanschette (1.1) eine schlauchwandfreie Struktur aufweist und über obere Enden der Bronchialstreben (1.2) mit einer Innenwand der Bronchialmanschette (1.1) verbunden ist, und wobei untere Enden der Bronchialstreben (1.2) mit der ringförmigen Öffnung (1.3) verbunden sind.

2. Schlauchwandfreier rechtsseitiger Doppellumen-Bronchialkatheter nach Anspruch 1, der ferner eine Haupttrachealmanschetten-Inflationsvorrichtung und eine Bronchialmanschetten-Inflationsvorrichtung (2.5) umfasst.

3. Schlauchwandfreier rechtsseitiger Doppellumen-Bronchialkatheter nach Anspruch 1 oder 2, wobei der Doppellumen-Beatmungskatheterkörper (2) ein Schlauch mit einer Zwei-Lumen-Struktur ist, wobei die linke Seite der Haupttrachealkatheter (2.1) ist und die rechte Seite der rechte Bronchialkatheter (2.2) ist.

4. Schlauchwandfreier rechtsseitiger Doppellumen-Bronchialkatheter nach Anspruch 1 oder 2, wobei sich die Haupttrachealmanschette (2.3) in dem unteren Abschnitt des Doppellumen-Beatmungskatheterkörpers (2) befindet, die Haupttrachealkatheteröffnung (2.4) sich unterhalb der Haupttrachealmanschette (2.3) befindet und die Haupttrachealkatheteröffnung (2.4) eine nach links unten gerichtete abgeschrägte Öffnung ist.

5. Schlauchwandfreier rechtsseitiger Doppellumen-Bronchialkatheter nach Anspruch 2, wobei die Haupttrachealmanschetten-Inflationsvorrichtung über einen parallel zum Doppellumen-Beatmungskatheterkörper (2) verlaufenden Haupttrachealmanschetten-Verbindungsschlauch (2.6) mit der Haupttrachealmanschette (2.3) verbunden ist.

6. Schlauchwandfreier rechtsseitiger Doppellumen-Bronchialkatheter nach Anspruch 2, wobei die Bronchialmanschetten-Inflationsvorrichtung (2.5) über einen parallel zum Doppellumen-Beatmungskatheterkörper (2) verlaufenden Bronchialmanschetten-Verbindungsschlauch (2.7) mit der Bronchialmanschette (1.1) verbunden ist.

## Revendications

1. Cathéter bronchique à double lumière côté droit sans paroi tubulaire comprenant :
- un corps de cathéter de ventilation à double lumière (2) comprenant un cathéter trachéal principal (2.1) et un cathéter bronchique droit (2.2),
- un ballonnet trachéal principal (2.3), et
- un corps de cathéter bronchique sans paroi tubulaire (1) constitué de :
- un ballonnet bronchique (1.1),
- des supports bronchiques (1.2), et
- une ouverture annulaire (1.3)
où l'extrémité inférieure du cathéter bronchique droit (2.2) est reliée au corps du cathéter bronchique sans paroi tubulaire (1), où un cathéter bronchique d'une partie inférieure du ballonnet bronchique (1.1) présente une structure sans paroi tubulaire et est relié à une paroi interne du ballonnet bronchique (1.1) par l'intermédiaire des extrémités supérieures des supports bronchiques (1.2), et
où les extrémités inférieures des supports bronchiques (1.2) sont reliées à l'ouverture annulaire (1.3).

2. Cathéter bronchique à double lumière côté droit sans paroi tubulaire selon la revendication 1, comprenant en outre un dispositif de gonflage du ballonnet trachéal principal et un dispositif de gonflage du ballonnet bronchique (2.5).

3. Cathéter bronchique à double lumière côté droit sans paroi tubulaire selon la revendication 1 ou 2, où le corps du cathéter de ventilation à double lumière (2) est un tube avec une structure à deux lumières, le côté gauche constituant le cathéter trachéal principal (2.1) et le côté droit le cathéter bronchique droit (2.2).

4. Cathéter bronchique à double lumière côté droit sans paroi tubulaire selon la revendication 1 ou 2, où le ballonnet trachéal principal (2.3) est situé dans la partie inférieure du corps du cathéter de ventilation à double lumière (2), l'ouverture du cathéter trachéal principal (2.4) est située sous le ballonnet trachéal principal (2.3), et l'ouverture du cathéter trachéal principal (2.4) est une ouverture biseautée orientée vers le bas à gauche.

5. Cathéter bronchique à double lumière côté droit sans paroi tubulaire selon la revendication 2, où le dispositif de gonflage du ballonnet trachéal principal est relié au ballonnet trachéal principal (2.3) via un tube de raccordement du ballonnet trachéal principal (2.6) parallèle au corps du cathéter de ventilation à double lumière (2).

6. Cathéter bronchique à double lumière côté droit sans paroi tubulaire selon la revendication 2, où le dispositif de gonflage du ballonnet bronchique (2.5) est relié au ballonnet bronchique (1.1) via un tube de raccordement du ballonnet bronchique (2.7) parallèle au corps du cathéter de ventilation à double lumière (2).
